Europäisches Patentamt

European Patent Office

Office européen des brevets

(11) Veröffentlichungsnummer: **0 285 756**
**A1**

(12) **EUROPÄISCHE PATENTANMELDUNG**

(21) Anmeldenummer: 88101448.4

(22) Anmeldetag: 02.02.88

(51) Int. Cl.⁴: **A61F 2/34**

(30) Priorität: 30.03.87 CH 1219/87

(43) Veröffentlichungstag der Anmeldung:
**12.10.88 Patentblatt 88/41**

(84) Benannte Vertragsstaaten:
**AT DE FR GB IT**

(71) Anmelder: **GEBRÜDER SULZER
AKTIENGESELLSCHAFT
Zürcherstrasse 9
CH-8401 Winterthur(CH)**

Anmelder: **Protek AG
Stadtbachstrasse 64
CH-3012 Bern(CH)**

(72) Erfinder: **Wagner, Heinz, Prof. Dr.
Orthopädische Klinik
Wichernhaus, Krankenhaus Rummelsberg
D-8501 Schwarzenbruck(DE)**

(74) Vertreter: **Dipl.-Ing. H. Marsch Dipl.-Ing. K.
Sparing Dipl.-Phys.Dr. W.H. Röhl
Patentanwälte
Rethelstrasse 123
D-4000 Düsseldorf(DE)**

(54) **Künstliche Hüftsgelenkspfanne.**

(57) In einer im Beckenknochen verankerbaren metallenen Aussenschale (1) ist ein Kunststoff-Pfannenkörper (11) dadurch verdrehbar gehalten, dass ein Ringflansch (15) auf der äusseren Oberfläche des Pfannenkörpers (11) durch einen Gewindering (16) auf einem Absatz (8) des Innenhohlraumes der Aussenschale (1) eingeklemmt ist.

Dieses Festklemmen des Pfannenkörpers (11) ermöglicht ein exaktes Einstellen eines beispielsweise bezüglich der Polachse (4) nicht rotationssymmetrisch gestalteten Pfannenkörpers (11) auf einen bestimmten Meridian der Aussenschale (1), d.h. in einer bestimmten Lage, z.B. relativ zur Frontalebene, des menschlichen Körpers.

Fig. 1

## Künstliche Hüftgelenkspfanne

Die Erfindung betrifft eine künstliche Hüftgelenkspfanne bestehend aus einer metallenen Aussenschale und aus einem, die Pfannenschale für die Aufnahme des Gelenkkopfes enthaltenden Kunststoff-Pfannenkörper, der in der Aussenschale fixierbar ist, wobei der Pfannenkörper mit einem aus seiner äusseren Oberfläche hervorspringenden Ringflansch auf einem Absatz des Innenhohlraumes der Aussenschale abgestüzt ist.

Eine Hüftgelenkspfanne der vorstehend genannten Art ist bekannt aus der FR-A-2.210.909; bei dieser Konstruktion wird zur Verbindung der beiden Pfannenelemente der Pfannenkörper in die Aussenschale eingeschraubt, wofür beide in ihren äquartorialen Bereichen mit entsprechenden Gewinden versehen sind. Da ein Gewinde mehr oder weniger stark "angezogen" werden kann, ist es nicht möglich, eine bestimmte Lage für den Pfannenkörper bei der Implantation exakt zu erreichen, beispielsweise bei Pfannenkörpern, die nicht rotationssymmetrisch ausgebildet sind und daher in einer bestimmten Lage, bezogen z.B. auf die Frontalebene des Körpers, eingebaut werden müssen.

Aufgabe der Erfindung ist es daher, eine Hüftgelenkspfanne zu schaffen, bei der der Pfannenkörper um die Polachse der Pfanne stufenlos verdrehbar eingebaut und fixiert werden kann, so dass bestimmte Punkte auf dem Umfang zu Bezugspunkten im menschlichen Körper, beispielsweise zur Frontalebene, exakt eine bestimmte Winkellage haben.

Diese Aufgabe wird mit der Erfindung dadurch gelöst, dass der Ringflansch mit Hilfe eines in den Innenhohlraum der Aussenschale eingeschraubten Gewinderinges eingeklemmt ist.

Da die Fixierung des Pfannenkörpers in der Aussenschale allein durch Einklemmen des Ringflansches erfolgt, kann der Pfannenkörper vor dem Einklemmen in der Aussenschale beliebig gedreht werden, so dass bei Pfannen, bei denen die Aussenschale beispielsweise rotationssymmetrisch zur Polachse ausgebildet ist, während der Pfannenkörper dazu unsymmetrisch gestaltet ist, die Unsymmetrie des Pfannenkörpers in jeder beliebigen "geographischen Länge" exakt fixiert werden kann.

Obwohl die Aussenschale vorzugsweise über ein Gewinde in den Beckenknochen eingeschraubt ist, ist es vorteilhaft, eine zusätzliche Fixierung mit Hilfe von Knochenschrauben vorzusehen. Um dem Operateur für das "Setzen" dieser Schrauben eine Anzahl Auswahlmöglichkeiten zu bieten, können in der Aussenschale mindestens zwei Reihen in Umfangsrichtung verteilter Bohrungen für den Durchtritt der Knochenschrauben vorgesehen sein. Im allgemeinen werden im konkreten Fall von diesen Bohrungen höchstens einzelne, beispielsweise zwei oder drei, "genutzt"; in die anderen wächst im Laufe der Zeit Knochengewebe ein.

Als Material für die Aussenschale dient vorteilhaft eines der in der Implantat-Technik üblichen Metalle oder Metall-Legierungen, beispielsweise Reintitan oder eine Titanlegierung. Der Gewindering ist im allgemeinen ebenfalls aus Metall gefertigt und besteht vorteilhafterweise aus dem gleichen Material wie die Aussenschale. Ein bevorzugter Kunststoff für den Pfannenkörper ist Polyäthylen mit einer für Implantate dieser Art üblichen Qualität und Spezifikation.

Im folgenden wird die Erfindung anhand eines Ausführungsbeispiels im Zusammenhang mit der Zeichnung erläutert.

Fig. 1 zeigt einen Schnitt I-I von Fig. 2 durch die Pfannenkonstruktion;

Fig. 2 gibt eine Aufsicht in Richtung der Polachse auf Aussenschale wieder.

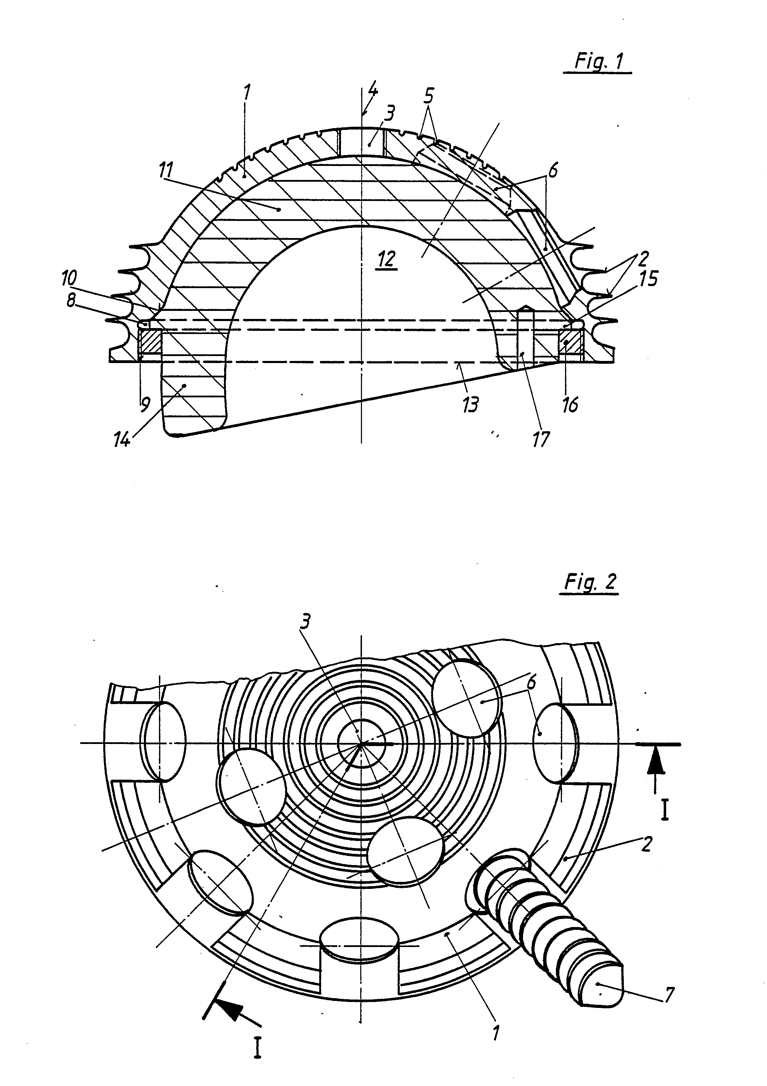

Die beispielsweise aus Titan bestehende Aussenschale 1 ist im Bereich niedriger "geographischer" Breiten mit einem Aussengewinde 2 versehen, mit dem sie in den Beckenknochen eingeschraubt wird. An ihrem Pol, durch den die gleichzeitig eine Rotationssymmetrieachse der Aussenchale bildende Polachse 4 verläuft, weist sie eine Gewindebohrung 3 auf, mit deren Hilfe ein nicht gezeigtes Setz-und Einschraubinstrument zentriert gehalten wird. Kreisringförmige Rillen 5 dienen in der Umgebung des Poles dem Ein-und Anwachsen von Gewebe. In zwei Reihen sind in der Aussenschale 1 über den Umfang verteilt Durchtrittsbohrungen 6 für Knochenschrauben 7 (Fig. 2) vorgesehen. Diese Bohrungen sind auf der Innenseite der Schale 1 so erweitert, dass die nicht gezeigten Köpfe der Schrauben 7 in der Aussenschale 1 versenkt werden können.

Am äquatorialen Rand hat der Innenhohlraum der Aussenschale 1 einen stufenförmigen Absatz 8, in dessen Wand ein Gewinde 9 eingeschnitten ist. Zur Vermeidung einer scharfen Kante am Uebergang in den Innenhohlraum ist eine Abschrägung 10 vorgesehen.

In der Aussenschale 1 ruht der Pfannenkörper 11 aus Kunststoff; er enthält die eigentliche Pfannenschale 12 für die Aufnahme eines nicht gezeigten Gelenkkopfes. Bei der dargestellten Ausführungsform ist der Pfannenkörper 11 auf einem Teil seines Umfanges über die Aequatorebene 13 hinaus verlängert. Die Verlängerung 14 weist bei der implantierten Pfanne nach lateral und hat die Aufgabe, Luxationen des Gelenkkopfes beim extremen Abbiegen des Hüftgelenkes zu vermeiden.

Aus der kugeligen, äusseren Oberfläche des Pfannenkörpers 11 springt ein Ringflansch 15 vor, mit dem der Pfannenkörper 11 im Absatz 8 abgestützt ist. Zum fixierenden Einklemmen des Ringflansches 15 ist in das Gewinde 9 ein Gewindering 16 eingeschraubt, der vorzugsweise aus dem gleichen Material beteht wie die Aussenschale 1.

Für den Eingriff eines Setzinstrumentes sind im Rand des Pfannenkörpers auf dem Umfang - schliesslich Bohrungen 17 verteilt.

Eine Implantation der neuen Hüftgelenkspfanne kann beispielsweise derart erfolgen, dass zunächst die Aussenschale mit ihrem Gewinde 2 durch ein in mindestens einige der Bohrungen 6 eingreifendes, in der Bohrung 3 zentriertes Einschraubinstrument in den vorbereiteten Beckenknochen eingeschraubt wird. Anschliessend wird die Aussenschale 1 gegebenenfalls zusätzlich mit Hilfe von Knochenschrauben 7 verankert. Mit einem Setzinstrument, das in die Bohrungen 17 eingreift, wird der Pfannenkörper 11 in der richtigen Lage der Verlängerung 14 dann in die Aussenschale 1 eingesetzt; abschliessend wird der Gewindering 16 durch ein mit dem Setzinstrument vorteilhafterweise kombiniertes Einschraubinstrument wie ein Ueberwurfmutter soweit angezogen, bis die erforderliche Klemmung für ein unverrückbares Festsitzen des Pfannenköpers 11 in der Aussenschale 1 erreicht ist.

**Ansprüche**

1. Künstliche Hüftgelenkspfanne bestehend aus einer metallenen Aussenschale und aus einem, die Pfannenschale für die Aufnahme des Gelenkkopfes enthaltenden Kunststoff-Pfannenkörper, der in der Aussenschale fixierbar ist, wobei der Pfannenkörper mit einem aus seiner äusseren Oberfläche hervorspringenden Ringflansch auf einem Absatz des Innenhohlraumes der Aussenschale abgestüzt ist, dadurch gekennzeichnet, dass der Ringflansch (15) mit Hilfe eines in den Innenhohlraum der Aussenschale (1) eingeschraubten Gewinderinges (16) eingeklemmt ist.

2. Hüftgelenkspfanne nach Anspruch 1, dadurch gekennzeichnet, dass, bezüglich der Polachse (4), die Aussenschale (1) rotationssymmetrisch, der Pfannenkörper (11) jedoch unsymmetrisch ausgebildet ist.

3. Hüftgelenkspfanne nach Anspruch 2, dadurch gekennzeichnet, dass die Aussenschale (1) mit mindestens zwei Reihen in Umfangsrichtung verteilter Bohrungen (6) versehen ist.

*Fig. 1*

*Fig. 2*

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 602 261 (G. SOSTEGNI) * Figur 7; Seite 9, Zeilen 13-25; Seite 10, Zeilen 1-8 * | 1 | A 61 F 2/34 |
| Y | | 2 | |
| Y | GB-A-2 058 575 (M.T. NORTON) * Figur 1 * | 2 | |
| A | US-A-3 740 769 (HABOUSH) | 3 | |
| A | US-A-4 695 282 (FORTE et al.) | 2 | |
| P,X | EP-A-0 253 941 (G. SOSTEGNI) * Figur 2; Spalte 3, Zeilen 43-54 * | 1 | |

**RECHERCHIERTE SACHGEBIETE (Int. Cl.4)**

A 61 F

Der vorliegende Recherchenbericht wurde für alle Patentansprüche erstellt

| Recherchenort | Abschlußdatum der Recherche | Prüfer |
|---|---|---|
| DEN HAAG | 11-07-1988 | ARGENTINI A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)